# EUROPÄISCHE PATENTSCHRIFT

(11) **EP 2 626 010 B1**
(45) Veröffentlichungstag und Bekanntmachung des Hinweises auf die Patenterteilung: **26.08.2015**
(21) Anmeldenummer: 12154820.0
(22) Anmeldetag: 10.02.2012
(51) Int. Cl.: A61B 17/00, A61C 1/08

(54) **Medizinisches, insbesondere dentales oder chirurgisches, Instrument mit Beschichtung**
Medical, in particular dental or surgical instrument with coating
Instrument médical, notamment dentaire ou chirurgical, avec revêtement

(43) Veröffentlichungstag der Anmeldung: 14.08.2013
(73) Patentinhaber: W & H Dentalwerk Bürmoos GmbH, 5111 Bürmoos (AT)
(72) Erfinder: Wagner, Beate, 2620 Neunkirchen (AT)
(74) Vertreter: Benda, Ralf

(56) Entgegenhaltungen:
- EP-A1- 0 517 244
- DE-A1- 19 828 976
- US-A- 5 527 340
- US-A1- 2004 091 750

## Beschreibung

Die vorliegende Erfindung betrifft eine Oberfläche eines medizinischen, insbesondere dentalen oder chirurgischen, Instruments mit einer darauf aufgebrachten oder abgeschiedenen Schicht, ein medizinisches, insbesondere dentales oder chirurgisches, Instrument mit einer derartigen Schicht und ein Verfahren zur Herstellung eines medizinischen, insbesondere dentalen oder chirurgischen, Instruments mit einer derartigen Schicht.

Aus der Patentanmeldung US 2004/0091750 A1 ist ein medizinisches Instrument bekannt, dessen Oberfläche zum Abweisen von Schmutz mit Kunststoff, insbesondere Teflon, beschichtet ist. Die Kunststoffschicht ist derart beschaffen, dass die Rauheit der Oberfläche erhalten bleibt, so dass ein Anwender das medizinische Instrument sicher halten kann. Durch die Teflonbeschichtung sollen somit also zwei gewünschte, einander oftmals ausschließende Eigenschaften einer Oberfläche eines medizinischen Instruments realisierbar sein, nämlich das Abweisen von Schmutz von der Oberfläche und das sichere, feste Halten des Instruments durch den Anwender.

Die Patentanmeldung DE 198 28 976 A1 offenbart ein elektrochirurgisches Gerät in Form einer Schere, deren beide Scherenschenkel durch eine Schwenkvorrichtung, die in einer Bohrung in den Scherenschenkel aufgenommen ist, verbunden sind. Um die beiden Scherenschenkel elektrisch voneinander zu isolieren, wird vorgeschlagen, die an den Bohrungswandungen anliegenden Flächen der Schwenkvorrichtung mit einer elektrisch isolierenden Glas-Keramik-Beschichtung zu versehen.

Ein Nachteil eines medizinischen Instruments, dessen Oberfläche mit einer Kunststoff-, insbesondere Teflonschicht, versehen ist, besteht darin, dass die Kunststoff- oder Teflonschicht gegenüber mechanischen Einwirkungen sehr empfindlich ist. Dies bedeutet in der Praxis, dass die Kunststoff- oder Teflonschicht des medizinischen Instruments schon nach kurzer Zeit beschädigt ist, zum Beispiel durch Abrieb oder durch Kontakt mit anderen Instrumenten, und ihre Wirksamkeit verliert oder verringert wird.

Der vorliegenden Erfindung liegt daher die Aufgabe zugrunde, eine Oberfläche eines medizinischen, insbesondere dentalen oder chirurgischen, Instruments oder Instrumententeils oder eine Beschichtung für eine Oberfläche eines medizinischen, insbesondere dentalen oder chirurgischen, Instruments oder Instrumententeils zu schaffen, die unter Beibehaltung der beiden im Vorstehenden genannten, gewünschten Eigenschaften (Abweisen von Schmutz von der Oberfläche und sicheres, festes Halten des Instruments durch den Anwender) gegenüber mechanischen Einflüssen weniger empfindlich ist.

Diese Aufgabe wird gemäß der vorliegenden Erfindung durch ein medizinisches, insbesondere dentales oder chirurgisches, Instrument oder Instrumententeil mit den Merkmalen des Anspruchs 1 und durch ein Verfahren zur Herstellung eines Instruments oder Instrumententeils mit den Merkmalen des Anspruchs 12 gelöst. Die Oberfläche des medizinischen, insbesondere dentalen oder chirurgischen, Instruments oder Instrumententeils, umfasst: Ein Substrat mit einer rauen Oberflächenstruktur sowie eine Schicht, die derart auf dem Substrat aufgebracht oder abgeschieden ist, dass die raue Oberflächenstruktur des Substrats durch die Schicht zumindest nicht vollständig geglättet ist oder die Rauheit erhöht ist, wobei die auf dem Substrat aufgebrachte oder abgeschiedene Schicht als glas-keramische Schicht ausgebildet ist, und wobei die Rauheit der auf dem Substrat aufgebrachten oder abgeschiedenen glas-keramischen schicht Ra = 0,5 - 1,8 µm und/oder Rz = 3 - 14 µm beträgt.

Als glas-keramische Schicht oder glas-keramische Substanz wird vorzugsweise ein Gemisch oder Verbundwerkstoff aus amorphem Glas oder einer amorphen, glas-ähnlichen Matrix und darin enthaltenen polymerisierten, vorzugsweise organischen, Komponenten verstanden. Die glas-keramische Schicht oder glas-keramische Substanz weist vorzugsweise eine amorphe Schichtstruktur auf. Gemäß einem Ausführungsbeispiel weist die auf dem Substrat aufgebrachte oder abgeschiedene glas-keramische Schicht Silizium, insbesondere Siliziumoxid und / oder zumindest eine (plasma-)polymerisierte, vorzugsweise zumindest teilweise organische, Siliziumverbindung, auf. Gemäß einem anderen Ausführungsbeispiel umfasst die glas-keramische Schicht oder glas-keramische Substanz eine Mischstruktur aus Siliziumdioxid und / oder amorphen, quarzähnlichen Schichten mit eingelagerten organischen, plasma-polymerisierten Komponenten.

Die Verwendung einer glas-keramischen Substanz oder Schicht zur Beschichtung einer Oberfläche eines medizinischen, insbesonder dentalen oder chirurgischen, Instruments oder Instrumententeils hat folgende Vorteile:
Die glas-keramische Schicht ist oder wird vorzugsweise derart dünn auf das Substrat aufgetragen oder darauf abgeschieden, dass die Rauheit des Substrats zumindest nicht vollständig geglättet ist, vorzugsweise im Wesentlichen erhalten bleibt, oder sogar etwas erhöht ist. Vorzugsweise folgt oder entspricht die glas-keramische Schicht also im Wesentlichen der Oberflächenstruktur des Substrats, insbesondere ohne Vertiefungen zu glätten oder diese erheblich oder vollständig auszufüllen. Bevorzugt beträgt die Schichtstärke der auf dem Substrat aufgebrachten oder abgeschiedenen glas-keramischen Schicht in etwa 1 - 8 µm, vorzugsweise 3 - 8 µm auf Instrumenten-Außenteilen, zum Beispiel einer Außenhülse, und 1 - 5 µm auf Instrumenten-Innenteilen, zum Beispiel einer Werkzeughaltevorrichtung oder einer Welle. Die derart im Wesentlichen bestehen gebliebene Rauheit des Substrats und / oder die derart entstandene oder übernommene Rauheit der auf dem Substrat aufgebrachten oder abgeschiedenen Schicht ermöglich dem Anwender das sichere, möglichst rutschfreie Halten des Instruments oder Instrumententeils.

Vorzugsweise beträgt die Rauheit des Substrats, auf welches die glas-keramische Schicht aufgetragen oder abgeschieden ist, in etwa Ra = 0,5 - 1,5 µm und / oder Rz = 3 - 12 µm.

Die glas-keramische Schicht ist oder wird vorzugsweise im Wesentlichen geschlossen oder porenfrei auf dem Substrat aufgetragen oder darauf abgeschieden und weist damit eine schmutzabweisende Eigenschaft und eine hohe Barriereeigenschaft, insbesondere gegenüber Gas- und Wasserdampfdiffusion, auf. Vorzugsweise ist oder wird die glas-keramische Schicht, insbesondere die Oberfläche der glas-keramischen Schicht, modifiziert, so dass die glas-keramische Schicht, insbesondere deren Oberfläche, hydrophobe Oberflächeneigenschaften erhält. Die Modifikation erfolgt zum Beispiel durch Einstellung oder Steuerung des Sauerstoffgehalts während des Beschichtungsprozesses (siehe unten). Die hydrophobe Oberflächeneigenschaft erhöht noch zusätzlich die schmutzabweisenden Eigenschaften der glas-keramischen Schicht. Falls gewünscht ist es auch möglich, die glas-keramische Schicht, insbesondere deren Oberfläche, derart zu modifizieren, dass sie hydrophile Oberflächeneigenschaften erhält.

Schließlich ist die glas-keramische Schicht im Vergleich zu der aus dem Stand der Technik bekannten Teflonbeschichtung mechanisch erheblich fester und damit gegenüber Abrieb und Kontakt mit scharfen Kanten oder Schneiden, zum Beispiel anderer medizinischer Instrumente, erheblich widerstandsfähiger. Die Härte oder mechanische Festigkeit der glas-keramischen Schicht beträgt zum Beispiel 600 - 800 HV, vorzugsweise etwa 700 HV.

Ein weiterer Vorteil der glas-keramischen Schicht ist, dass diese vorzugsweise im Wesentlichen chemisch inert ist. Die glas-keramische Schicht ist damit unter anderem auch im Wesentlichen korrosionsbeständig, insbesondere gegenüber aggressiven Reinigungsmedien und Wasserdampf.

Des Weiteren ist die glas-keramische Schicht vorzugsweise derart ausgebildet, dass sie Temperaturveränderungen von in etwa 100°C - 200°C widersteht, so dass sie bevorzugt bekannten Reinigungs- oder Sterilisationsverfahren mit derartigen Temperaturveränderungen, insbesondere zumindest mehrere hunderte Male, problemlos ausgesetzt werden kann. Insbesondere weist die glas-keramische Schicht einen sehr geringen Wärmeausdehnungskoeffizient in unterschiedlichen Temperaturbereichen auf, so dass dadurch ein Bruch durch Temperaturschock vermieden wird. Der Wärmeausdehnungskoeffizient beträgt zum Beispiel zumindest 0,55 · 10⁻⁶ K⁻¹.

Gemäß einem Ausführungsbeispiel ist die auf dem Substrat aufgebrachte oder abgeschiedene glas-keramische Schicht transparent. Damit ist es in vorteilhafter Weise möglich, geometrische und / oder alphanummerische Strukturen oder Formen, zum Beispiel Kanten, Rändelungen, Schriftzeichen, Zahlen, Marken, Logos, Benutzungs- oder Warnhinweise, auf dem Instrument oder Instrumententeil zu formen, so dass diese aufgrund der Transparenz der glas-keramischen Schicht für den Anwender erkennbar oder sichtbar sind.

In vorteilhafter Weise ist es möglich, die glas-keramische Schicht wahlweise auf ein durch ein Metall gebildetes Substrat, insbesondere ein Substrat aus Stahl oder Messing, oder auf ein durch Kunststoff gebildet Substrat aufzubringen oder aufzutragen. Gemäß einem Ausführungsbeispiel ist zwischen dem Substrat oder der Oberfläche des Instruments oder Instrumententeils und der glas-keramischen Schicht eine Zwischenschicht oder Haftvermittlerschicht vorgesehen, zum Beispiel eine Metalllegierung, bevorzugt eine Nickel-Chrom-Schicht. Gemäß einem alternativen Ausführungsbeispiel ist die glas-keramischen Schicht direkt (ohne Haftvermittler- oder Zwischenschicht) auf dem Substrat oder der Oberfläche des Instruments oder Instrumententeils aufgebracht oder abgeschieden.

Vorzugsweise ist die glas-keramische Schicht durch ein CVD (chemical-vapor-deposition) - Verfahren unter Bildung eines Plasmas auf dem Substrat oder der Oberfläche abgeschieden oder aufgebracht. Bei diesem, grundsätzlich bekannten, durch Plasma unterstützten CVD-Verfahren wird das Gas und / oder darin enthaltene, abzuscheidende Komponenten mit Hilfe einer Hochfrequenzspannung in den Plasmazustand versetzt. Das Gas oder die abzuscheidenden Komponenten sind im Plasmazustand sehr energiereich und scheiden sich anschließend auf dem Substrat oder der Oberfläche ab. Durch entsprechende Wahl der Prozessparameter des Verfahrens, zum Beispiel der Höhe oder Dauer des Energieeintrags zur Bildung des Plasmas, des Gasdrucks oder des Sauerstoffgehalts im Plasmagas, ist es möglich, die Abscheidung einer glas-keramischen Schicht zu erzielen.

Insbesondere enthält das Gas silizium-organische Verbindungen oder Silangas und wird der Sauerstoffgehalt im Plasmagas derart gewählt, dass der Sauerstoff nicht ausreicht, die silizium-organische Verbindungen oder Silangas (vollständig) in Siliziumdioxid, Kohlendioxid und Wasser umzusetzen, wodurch die glas-keramischen Schicht, insbesondere mit ihrer amorphen, quarzähnlichen Struktur mit eingelagerten organischen, plasma-polymerisierten Komponenten, entsteht. Durch das Plasma unterstützte CVD-Verfahren, insbesondere durch die (geringe oder unterstöchiometrische) Sauerstoffkonzentration, wird somit vorzugsweise zumindest die quantitativ zu große Bildung von Kristallen, insbesondere von Siliziumdioxid, verhindert und erst dadurch die Entstehung oder Abscheidung einer, insbesondere geschlossenen oder durchgängigen, glas-keramischen Schicht möglich.

Ein Instrument oder Instrumententeil, welches mit der glas-keramischen Schicht versehen ist, kann zum Beispiel als Handelement, Handgriff, Handstück, Winkelstück, Adapter, Antriebseinheit, Kupplungsvorrichtung, Luft- oder Elektromotor oder als Teil davon ausgebildet sein. Das Instrument oder Instrumententeil kann selbstverständlich unterschiedlichste Formen aufweisen, zum Beispiel gerade, gebogen, abgewinkelt, pistolenförmig, es kann ein- oder mehrteilig sein und es kann dazu ausgebildet sein, mechanische und / oder elektrische Energie und / oder zumindest ein Fluid abzugeben und / oder aufzunehmen und / oder weiterzuleiten. Gemäß einem bevorzugten Ausführungsbeispiel umfasst das medizinische, insbesondere dentale oder chirurgische, Instrument oder Instrumententeil, welches mit der glas-keramischen Schicht versehen ist, eine Medienabgabevorrichtung zur Abgabe eines Mediums in Richtung einer Behandlungsstelle, insbesondere zur Abgabe eines Fluids und / oder elektromagnetischer Energie, und / oder eine durch eine (elektrische oder fluidbetriebene) Antriebsvorrichtung in Bewegung versetzbare Werkzeughalterung für ein auf eine Behandlungsstelle einwirkendes Werkzeug. Gemäß einem weiteren bevorzugten Ausführungsbeispiel ist das Instrument oder Instrumententeil, welches mit der glas-keramischen Schicht versehen ist, als aktives medizinisches Instrument oder Instrumententeil ausgebildet, also als medizinisches Instrument oder Instrumententeil, dessen Betrieb zumindest teilweise auf eine elektrische Energiequelle oder auf eine andere Energiequelle als die unmittelbar durch den menschlichen Körper oder die Schwerkraft erzeugte Energie angewiesen ist, zum Beispiel auf eine Fluid, insbesondere Druckluft, zur Verfügung stellende Energiequelle.

Als Oberfläche oder Substrat mit einer glas-keramischen Schicht sind erfindungsgemäß nicht nur Oberflächen oder Substrate an der Außenseite, an der Außenhülle oder am Außenumfang eines medizinischen Instruments oder Instrumententeils zu verstehen, sondern selbstverständlich auch Oberflächen oder Substrate im Inneren eines derartigen Instruments oder Instrumententeils oder Oberflächen oder Substrate an Kupplungs-, Verbindungs-, Trenn- oder Kontaktflächen eines medizinischen Instruments oder Instrumententeils. Im Inneren eines medizinischen Instruments oder Instrumententeils angeordnete Bauteile mit einer glas-keramischen Schicht können zum Beispiel zumindest Teile einer Welle, einer Werkzeughaltevorrichtung, eines Lagers oder einer Medien- oder Fluidleitung umfassen, die insbesondere schmutzabweisend oder korrosionsbeständig sein sollen.

Das erfindungsgemäße Verfahren zur Herstellung eines medizinischen, insbesondere dentalen oder chirurgischen, Instruments oder Instrumententeils nach Anspruch 1 ist in Anspruch 12 offenbart.

Vorzugsweise weist die durch das im Vorstehenden definierte Herstellungsverfahren auf dem Substrat aufgebrachte oder abgeschiedene glas-keramische Schicht Silizium, insbesondere Siliziumoxid und / oder zumindest eine (plasma-)polymerisierte, vorzugsweise zumindest teilweise organische, Siliziumverbindung, auf.

Vorzugsweise ist die durch das im Vorstehenden definierte Herstellungsverfahren auf dem Substrat aufgebrachte oder abgeschiedene glas-keramische Schicht transparent und weist eine hydrophobe Oberflächeneigenschaft auf.

Vorzugsweise wird die auf dem Substrat aufgebrachte oder abgeschiedene glas-keramische Schicht durch ein CVD (chemical-vapor-deposition) - Verfahren unter Bildung eines Plasmas auf der Oberfläche oder dem Substrat abgeschieden.

Die Erfindung wird nachfolgend anhand eines bevorzugten Ausführungsbeispiels und Bezug nehmend auf die beigefügten Zeichnungen erläutert:
Die Figuren 1 - 4 zeigen vier Ausführungsbeispiele medizinischer, insbesondere dentaler oder chirurgischer, Instrumente oder Instrumententeile, mit zumindest einer Oberfläche mit einer glas-keramischen Schicht.
Figur 5 zeigt ein erstes Ausführungsbeispiel einer glas-kermischen Schicht eines medizinischen, insbesondere dentalen oder chirurgischen, Instruments oder Instrumententeils.
Figur 6 zeigt ein zweites Ausführungsbeispiel einer glas-kermischen Schicht eines medizinischen, insbesondere dentalen oder chirurgischen, Instruments oder Instrumententeils.

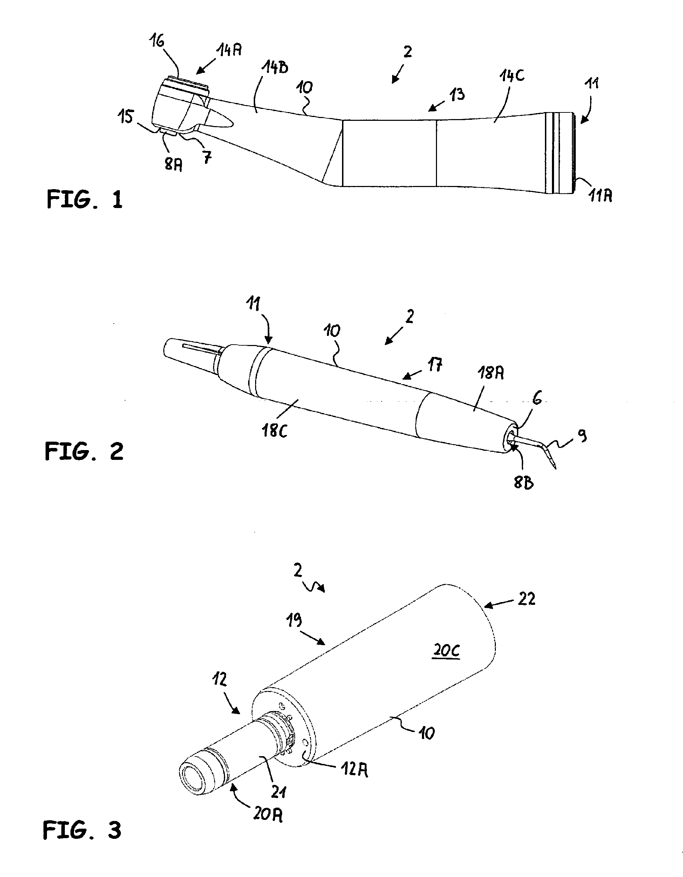

Die Figur 1 zeigt ein erstes Ausführungsbeispiel eines medizinischen, insbesondere dentalen oder chirurgischen, Instruments oder Instrumententeils 2 in Form eines Handgriffelements, insbesondere eines gebogenen oder gewinkelten Handstücks oder Winkelstücks 13. In der Figur 2 ist ein zweites Ausführungsbeispiel eines medizinischen, insbesondere dentalen oder chirurgischen, Instruments oder Instrumententeils 2 in Form eines Handgriffelements, insbesondere eines geraden Handstücks 17, vorzugsweise zur Entfernung von Zahnstein, dargestellt. Aufgrund vieler gleicher oder ähnlicher Bauteile werden die beiden Handgriffelemente 13, 17 im Folgenden gemeinsam beschrieben:
Die Instrumente 2 umfassen ein Kopfteil 14A, 18A, vorzugsweise ein daran anschließendes Halsteil 14B, und ein damit verbundenes Hauptteil 14C, 18C. Das Hauptteil 14C des Winkelstücks 13 ist gewinkelt zum Halsteil 14B angeordnet, wohingegen die beiden Teile 18A, 18C des Handstücks 17 gerade, im Wesentlichen entlang einer gemeinsamen Mittelachse angeordnet sind. Die Teile 14A - 14C und 18A, 18C weisen eine ein- oder mehrteiligen Außenhülse 10 auf.

Am oder im Kopfteil 14A, 18A ist eine, vorzugsweise lösbare, Werkzeughalterung 8A, 8B zur Aufnahme oder Halterung eines Behandlungswerkzeugs 9 vorgesehen. Die Werkzeughalterung 8A, 8B und das Behandlungswerkzeug 9 sind vorzugsweise in eine Arbeitsbewegung versetzbar, zum Beispiel in eine Rotations-, eine Hub- oder eine Schwingbewegung. Die Werkzeughalterung 8A, 8B ist zum Beispiel als reibschlüssige oder formschlüssige Verbindungsvorrichtung oder als Schraubverbindung ausgebildet.

Vorzugsweise ist am Kopfteil 14A, 18A der Instrumente 2, insbesondere an der Werkzeughalterung 8A, 8B oder um die Werkzeughalterung 8A, 8B und / oder um eine Werkzeugaufnahmeöffnung 15 der Außenhülse 10, alternativ am Halsteil 14B, eine erste Medienabgabevorrichtung 7 zur Abgabe zumindest eines Mediums, insbesondere von Luft und / oder Wasser, vorgesehen. Die erste Medienabgabevorrichtung 7 umfasst zum Beispiel eine oder mehrere Leitungen, Öffnungen und / oder Düsen, von denen ein Medium in Richtung der Behandlungsstelle und / oder des Werkzeugs abgebbar ist, oder eine Öffnung zur Verbindung mit einer Fluidbohrung im Werkzeug 9.

Vorzugsweise ist am Kopfteil 14A, 18A, insbesondere um die Werkzeughalterung 8A, 8B und / oder um die Werkzeugaufnahmeöffnung 15 der Außenhülse 10, alternativ am Halsteil 14B, eine Medienabgabevorrichtung in Form einer Lichtabgabevorrichtung 6 zur Abgabe von Licht in Richtung der Behandlungsstelle vorgesehen, zum Beispiel ein Lichtleiter und / oder eine Lichtquelle, vorzugsweise eine Leuchtdiode.

Die Instrumente 2, insbesondere das Winkelstück 13, umfassen des Weiteren vorzugsweise eine Werkzeuglösevorrichtung zum Lösen des Werkzeugs aus der Werkzeughalterung 8A. Die Werkzeuglösevorrichtung ist zum Beispiel mittels eines für den Anwender von außen zugänglichen Betätigungselements 16, insbesondere mittels eines Druckknopfs oder einer Drucktaste, bedienbar. Das Betätigungselement 16 ist bevorzugt am Kopfteil 14A vorgesehen, vorzugsweise im Wesentlichen gegenüber der Werkzeugaufnahmeöffnung 15.

Die Werkzeughalterung 8A, 8B und das Behandlungswerkzeug 9 sind wahlweise mittels einer in den Instrumenten 2 vorgesehenen Antriebsvorrichtung oder mittels einer von den Instrumenten 2 lösbaren, separaten Antriebseinheit in Bewegung versetzbar. Die in den Instrumenten 2 vorgesehene Antriebsvorrichtung umfasst zum Beispiel ein durch ein Fluid, insbesondere Druckluft, antreibbares Drehteil, insbesondere ein im Kopfteil 14A drehbar angeordnetes Laufrad, ein Flügelrad oder eine Drehhülse, oder eine elektrisch betreibbare Antriebsvorrichtung, zum Beispiel einen Elektromotor oder einen piezo-elektrischen oder magnetostriktiven Antrieb. Die von den Instrumenten 2 lösbare, separate Antriebseinheit umfasst zum Beispiel eine Motoreinheit, zum Beispiel einen Elektromotor oder einen Lamellen- oder Luftmotor (siehe Fig. 3).

Zur lösbaren Verbindung oder Kupplung der Instrumente 2 mit der lösbaren, separaten Antriebseinheit und / oder mit einer Steuer- oder Regeleinheit ist an dem Hauptteil 14C, 18C ein Anschluss- oder Kupplungsvorrichtung 11 vorgesehen. Die Anschlussvorrichtung 11 umfasst eine Kontaktfläche 11A, die eine Gegenkontaktfläche der lösbaren, separaten Antriebseinheit kontaktiert, wenn die Instrumente 2 mit der Antriebseinheit und / oder mit einer Steuer- oder Regeleinheit verbunden sind, und die frei liegt, wenn die Instrumente 2 von der Antriebseinheit und / oder mit einer Steuer- oder Regeleinheit getrennt sind. Die Anschlussvorrichtung 11 ist zum Beispiel als Steckverbindung, Schraubverbindung, Bajonettverbindung oder als Drehkupplung ausgebildet. Vorzugsweise ist die Anschlussvorrichtung 11 zur Übertragung zumindest eines Mediums und / oder von Daten ausgebildet, zum Beispiel zur Übertragung eines Fluids, insbesondere von Wasser oder Luft, von elektromagnetischer Strahlung, elektrischer Energie und / oder von elektrischen Signalen. Dazu sind an der Anschlussvorrichtung 11 und selbstverständlich auch in entsprechender Weise an der lösbaren, separaten Antriebseinheit und / oder Steuer- oder Regeleinheit eine oder mehrere elektrische Leitungen, Fluidleitungen, elektrische Kontakte, optische Leiter und / oder elektrische, optische oder Fluid-Verbindungselemente vorgesehen.

Im Halsteil 14B und / oder im Hauptteil 14C, 18C sind vorzugsweise Bauteile zur Übertragung eine Antriebsbewegung und / oder zur Leitung eines Mediums und / oder von Daten und / oder von elektrischen Signalen ausgebildet, zum Beispiel eine oder mehrere, in Bewegung versetzbare Wellen, eine Schwingachse, ein Getriebe, eine oder mehrere Leitungen oder Kanäle für Fluid, ein Lichtleiter oder elektrische Leitungen zur Übertragung elektrischer Signale oder Daten.

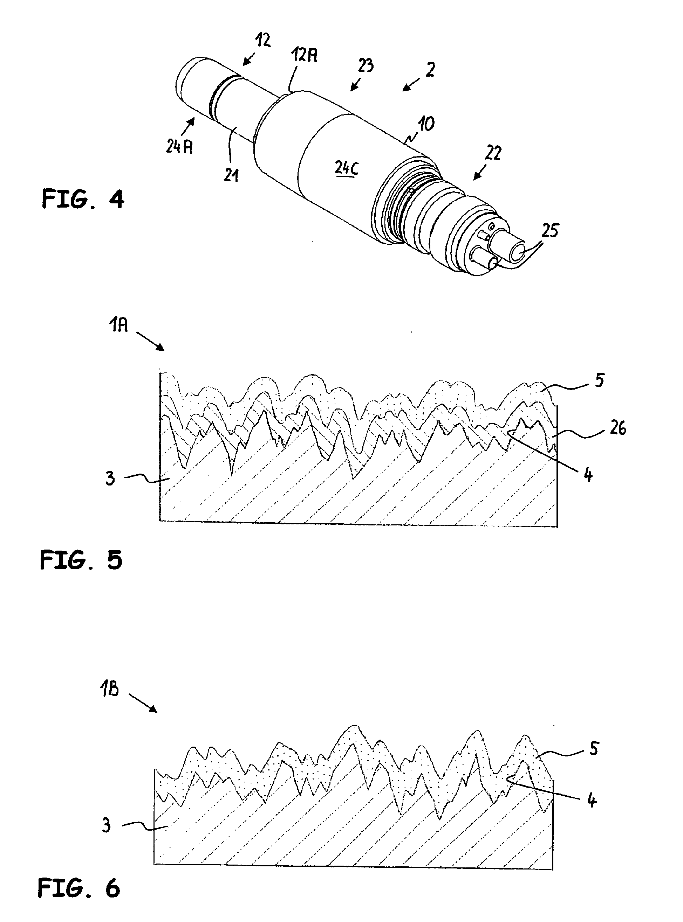

Die Figur 3 zeigt ein medizinisches, insbesondere dentales oder chirurgisches, Instrument oder Instrumententeil 2 in Form einer Antriebseinheit 19 zur Erzeugung einer Antriebsbewegung. Die Antriebseinheit 19 ist insbesondere als Motoreinheit ausgebildet, vorzugsweise als Elektromotor oder Lamellen- oder Luftmotor. Die Figur 4 zeigt ein medizinisches, insbesondere dentales oder chirurgisches, Instrument oder Instrumententeil 2 in Form eines Adapters oder einer Kupplung 23. Aufgrund vieler gleicher oder ähnlicher Bauteile werden die beiden Instrumente 2, i.e. die Antriebseinheit 19 und die Kupplung 23, im Folgenden gemeinsam beschrieben:
Beide Instrumente 2 umfassen je eine, ein- oder mehrteilige, Außenhülse 10. Die Instrumente 2 bzw. ihre Außenhülsen 10 weisen ein Hauptteil 20C, 24C und ein mit dem Hauptteil 20C, 24C verbundenes Anschlussteil 20A, 24A auf, vorzugsweise in Form einer Kupplungsvorrichtung 12. Mittels des Anschlussteils 20A, 24A sind die Instrumente 2 mit einem Werkzeug verbindbar, so dass die von der Antriebseinheit 19 erzeugte Antriebsbewegung und / oder zumindest ein Medium, insbesondere eine Fluid und / oder elektromagnetische Strahlung, auf das Werkzeug übertragbar ist / sind. Das Anschlussteil 20A, 24A ist zum Beispiel als Steckverbindung, Schraubverbindung, Bajonettverbindung oder als Drehkupplung ausgebildet.

Die Kupplungsvorrichtung 12 umfasst zum Beispiel ein Kupplungsrohr oder einen Kupplungszapfen 21 und eine Kontakt- oder Kupplungsfläche 12A. Die Kupplungsfläche 12A ist ausgebildet, eine Gegenkontaktfläche eines mit der Antriebseinheit 19 oder der Kupplung / dem Adapter 23 lösbar verbindbaren Instruments zu kontaktieren, zum Beispiel die Gegenkontaktfläche 11A des Handstücks 13, wenn die Antriebseinheit 19 oder die Kupplung / der Adapter 23 mit dem Instrument verbunden ist. Die Kupplungsfläche 12A liegt frei, wenn das Instrument von der Antriebseinheit 19 oder der Kupplung / dem Adapter 23 getrennt ist.

Im Hauptteil 20C der Antriebseinheit 19 sind der Motor oder zumindest ein Großteil der Motorkomponenten angeordnet, zum Beispiel der Rotor, der Stator, ein Steuer- oder Regelelement für den Motor, elektrische oder Fluid-Versorgungsleitungen zum Antrieb und / oder zur Kühlung des Motors, ein oder mehrere Sensoren zur Betriebsüberwachung des Motors, eine Rotorwelle, etc. Im Hauptteil 24C der Kupplung / des Adapters 23 kann gemäß einem bevorzugten Ausführungsbeispiel ein elektrodynamischer Wandler (Generator) angeordnet sein, der elektrische Energie zur Versorgung eines elektrischen Verbrauchers in der Kupplung / dem Adapter 23 und / oder in einem damit verbindbaren Instrument, zum Beispiel dem Handstücks 13, bereit stellt.

Gemäß einem Ausführungsbeispiel ist im Kupplungszapfen 21, insbesondere im Kupplungszapfen 21 der Antriebseinheit 19, eine Welle, zum Beispiel zumindest eine Teil der Rotorwelle oder eine mit der Rotorwelle verbundene Welle, und / oder ein Mitnehmer zum Übertragen oder Weiterleiten einer Antriebsbewegung, insbesondere der von der Antriebseinheit 19 erzeugten Antriebsbewegung, angeordnet.

An dem der Kupplungsvorrichtung 12 entgegen gesetzten Ende der Instrumente 2 oder an dem jeweiligen freien Ende des Hauptteils 20C, 24C ist eine, vorzugsweise lösbare, Verbindungsvorrichtung 22 zur Verbindung der Instrumente 2 mit einer Versorgungs- und / oder Steuer- oder Regeleinheit vorgesehen. Die Versorgungs- und / oder Steuer- oder Regeleinheit versorgt die Antriebseinheit 19, insbesondere deren Motor, oder die Kupplung / den Adapter 23, insbesondere deren Generator, zum Beispiel mit einem Antriebsmedium und / oder einem Kühlmedium und / oder elektrischen Signalen und / oder erhält vorzugsweise von der Antriebseinheit 19 oder der Kupplung / dem Adapter 23 zumindest ein elektrisches Signal oder Daten. Die Verbindungsvorrichtung 22 umfasst demgemäß vorzugsweise eine oder mehrere Medienübertragungselemente, zum Beispiel elektrische Leitungen zur Energieversorgung und / oder zum Datenaustausch, Fluidleitungen 25, optische Leiter, elektrische Kontakte und / oder elektrische, optische oder Fluid-Verbindungselemente.

Vorzugsweise sind die Antriebseinheit 19 und die Kupplung / der Adapter 23 dazu ausgebildet, zumindest ein Medium, zum Beispiel ein Fluid, insbesondere Wasser oder Druckluft, elektrische Energie, ein elektrisches Signal oder elektromagnetische Strahlung, insbesondere Licht, an ein mit der Antriebseinheit 19 oder der Kupplung / dem Adapter 23 verbundenes oder verbindbares Werkzeug oder Instrument, zum Beispiel Handstück 13, oder von einem mit der Antriebseinheit 19 oder der Kupplung / dem Adapter 23 verbundenen oder verbindbaren Werkzeug oder Instrument, zum Beispiel Handstück 13, zu übertragen. Dazu sind vorzugsweise an dem Anschlussteil 20A, 24A, insbesondere an der Kupplungsfläche 12A und / oder an dem Kupplungszapfen 21, ein oder mehrere Medienübertragungselemente vorgesehen, zum Beispiel elektrische Leitungen, Fluidleitungen, optische Leiter, elektrische Kontakte und / oder elektrische, optische oder Fluid-Verbindungselement. Zur Versorgung der Medienübertragungselemente des Anschlussteils 20A, 24A sind diese mit den Medienübertragungselementen der Verbindungsvorrichtung 22 über die Antriebseinheit 19 oder die Kupplung / den Adapter 23 durchsetzende elektrische, optische und / oder Fluid-Leitungen verbunden.

Zumindest ein Bauteil oder eine Oberfläche 1A, 1B eines Bauteils der im Vorstehenden beschriebenen Instrumente 2, 13, 17, 19, 23 der Figuren 1 - 4 ist mit einer glas-keramischen Schicht 5 versehen, zum Beispiel zumindest ein Teil der Außenhülse 10 und / oder der Anschlussvorrichtung 11, insbesondere deren Kontaktfläche 11A, und / oder der Kupplungsvorrichtung 12, insbesondere deren Kontaktfläche 12A, und / oder des Betätigungselements 16 und / oder der Werkzeughalterung 8A, 8B. Die Figuren 5 und 6 zeigen zwei Ausführungsbeispiele einer derartigen glas-keramischen Schicht 5, die wahlweise auf einer Oberfläche 1A, 1B zumindest eines Bauteils der im Vorstehenden beschriebenen Instrumenten 2, 13, 17, 19, 23 der Figuren 1-4 aufgebracht oder abgeschieden sein können.

Die Oberfläche 1A gemäß Figur 5 umfasst ein Substrat, zum Beispiel ein Metall oder einen Kunststoff. Das Substrat wird zum Beispiel durch die Außenhülse 10 oder deren äußerste Schicht gebildet. Selbstverständlich kann das Substrat jedoch auch jedes andere beliebige Bauteil des Instruments 2 umfassen. Das Substrat 3 weist eine raue Oberfläche oder Oberflächenstruktur 4 auf, dies ist in der Figur 5 durch den gezackten, berg- und talartigen Verlauf der Oberfläche des Substrats 3 dargestellt. Auf dem Substrat 3 ist eine Zwischenschicht oder eine Haftvermittlerschicht 26 vorgesehen. Die Zwischenschicht 26 umfasst gemäß einem Ausführungsbeispiel eine metallische Schicht, zum Beispiel eine Metalllegierung, insbesondere eine Nickel-Chrom-Schicht. Die Zwischenschicht 26 weist vorzugsweise eine Dicke von etwa 2 µm - 5 µm auf.

Auf der Zwischenschicht 26 ist die glas-keramische Schicht 5 angeordnet. Wie aus der Figur 5 zu erkennen ist, folgt die glas-keramische Schicht 5, genauso wie die Zwischenschicht 26, im Wesentlichen der rauen Oberfläche oder Oberflächenstruktur 4 des Substrats 3. Die raue Oberflächenstruktur 4 des Substrats 3 ist somit durch die glas-keramische Schicht 5 zumindest nicht vollständig geglättet, so dass dem Anwender ein sicheres, möglichst rutschfreies Halten des Instruments oder Instrumententeils 2 möglich ist. Aus der Figur 5 ist auch zu erkennen, dass die glas-keramische Schicht 5 im Wesentlichen homogen oder gleichmäßig auf dem Substrat 3 oder der Zwischenschicht 26 aufgebracht oder abgeschieden ist, so dass eine dichte, gleichmäßige, porenfreie und damit schmutzabweisende Oberflächenbeschichtung vorhanden ist. Die glas-keramische Schicht 5 weist zum Beispiel eine Dicke von etwa 1 µm - 8 µm auf, bevorzugt eine Dicke von in etwa 3,5 µm - 6 µm, insbesondere eine Dicke von etwa 4 µm. Wie im Vorstehenden bereits ausführlich beschrieben umfasst die glas-keramische Schicht 5 ein Gemisch aus amorphem Glas oder einer amorphen, glas-ähnlichen Matrix und darin enthaltenen (plasma)polymerisierten, vorzugsweise organischen, Komponenten. Insbesondere weist die glas-keramische Schicht 5 Silizium, insbesondere Siliziumoxid / Siliziumdioxid und / oder zumindest eine (plasma-)polymerisierte, vorzugsweise zumindest teilweise organische, Siliziumverbindung, auf.

Die in der Figur 6 dargestellte Oberfläche 1 B entspricht in ihrem Aufbau und in ihren Eigenschaften im Wesentlichen der Oberfläche 1A der Figur 5, so dass zur Vermeidung von Wiederholungen auf die diesbezügliche Beschreibung verwiesen wird. Die Oberfläche 1 B weist jedoch im Gegensatz zur Oberfläche 1A keine Zwischenschicht 26 auf: Es ist somit die glas-keramische Schicht 5 direkt auf dem Substrat 3, also zum Beispiel auf der Außenhülse 10 des Instruments 2, angeordnet. Das direkte Aufbringen oder Abscheiden der glas-keramischen Schicht 5 ohne Zwischenschicht ist sowohl bei einem metallischen Substrat als auch bei einem Substrat aus Kunststoff möglich.

Alle Merkmale aller beschriebenen und dargestellten Ausführungsbeispiele sind miteinander kombinierbar.

## Patentansprüche

1. Medizinisches, insbesondere dentales oder chirurgisches, Instrument oder Instrumententeil (2) mit einer Oberfläche (1A, 1B) umfassend ein Substrat (3) mit einer rauen Oberflächenstruktur (4) sowie eine Schicht, die derart auf dem Substrat (3) aufgebracht oder abgeschieden ist, dass die raue Oberflächenstruktur (4) des Substrats (3) durch die Schicht zumindest nicht vollständig geglättet ist oder die Rauheit erhöht ist, wobei die auf dem Substrat (3) aufgebrachte oder abgeschiedene Schicht als glas-keramische Schicht (5) ausgebildet ist und wobei die Rauheit der auf dem Substrat (3) aufgebrachten oder abgeschiedenen glas-keramischen Schicht (5) Ra = 0,5 - 1,8 µm und / oder Rz = 3 - 14 µm beträgt.

2. Medizinisches, insbesondere dentales oder chirurgisches, Instrument oder Instrumententeil (2) nach Anspruch 1, **dadurch gekennzeichnet, dass** die Rauheit des Substrats (3) in etwa Ra = 0,5 - 1,5 µm und / oder Rz = 3 - 12 µm beträgt.

3. Medizinisches, insbesondere dentales oder chirurgisches, Instrument oder Instrumententeil (2) nach Anspruch 1 oder 2, **dadurch gekennzeichnet, dass** die Härte oder mechanische Festigkeit der glas-keramischen Schicht (5) 600 - 800 HV beträgt.

4. Medizinisches, insbesondere dentales oder chirurgisches, Instrument oder Instrumententeil (2) nach einem der vorstehenden Ansprüche, **gekennzeichnet durch** eine auf dem Substrat (3) aufgebrachte oder abgeschiedene glas-keramische Schicht (5) mit hydrophober Oberflächeneigenschaft.

5. Medizinisches, insbesondere dentales oder chirurgisches, Instrument oder Instrumententeil (2) nach einem der Ansprüche 1 - 3, **gekennzeichnet durch** eine auf dem Substrat (3) aufgebrachte oder abgeschiedene glas-keramische Schicht (5) mit hydrophiler Oberflächeneigenschaft.

6. Medizinisches, insbesondere dentales oder chirurgisches, Instrument oder Instrumententeil (2) nach einem der vorstehenden Ansprüche, **dadurch gekennzeichnet, dass**
die Schichtstärke der auf dem Substrat (3) aufgebrachten oder abgeschiedenen glas-keramischen Schicht (5) in etwa 1 - 8 µm beträgt.

7. Medizinisches, insbesondere dentales oder chirurgisches, Instrument oder Instrumententeil (2) nach einem der Ansprüche 1 - 6, **gekennzeichnet durch** eine auf dem Substrat (3) aufgebrachte oder abgeschiedene transparente glas-keramische Schicht (5).

8. Medizinisches, insbesondere dentales oder chirurgisches, Instrument oder Instrumententeil (2) nach einem der Ansprüche 1 - 7, **dadurch gekennzeichnet, dass** die auf dem Substrat (3) aufgebrachte oder abgeschiedene glas-keramische Schicht (5) Silizium, insbesondere Siliziumoxid und / oder zumindest eine polymerisierte, vorzugsweise zumindest teilweise organische, Siliziumverbindung, aufweist.

9. Medizinisches, insbesondere dentales oder chirurgisches, Instrument oder Instrumententeil (2) nach einem der Ansprüche 1 - 8, **dadurch gekennzeichnet, dass** das Substrat (3) durch ein Metall, insbesondere aus Stahl oder Messing, oder durch Kunststoff gebildet ist.

10. Medizinisches, insbesondere dentales oder chirurgisches, Instrument oder Instrumententeil (2) nach einem der Ansprüche 1 - 9, **dadurch gekennzeichnet, dass** die glas-keramische Schicht (5) auf dem Substrat (3) durch ein CVD (chemical-vapor-deposition) - Verfahren unter Bildung eines Plasmas aufgebracht oder abgeschieden ist.

11. Medizinisches, insbesondere dentales oder chirurgisches, Instrument oder Instrumententeil (2) nach einem der vorstehenden Ansprüche, **gekennzeichnet durch** eine Medienabgabevorrichtung (6, 7) zur Abgabe eines Mediums in Richtung einer Behandlungsstelle, insbesondere zur Abgabe eines Fluids und / oder elektromagnetischer Energie, und / oder eine **durch** eine Antriebsvorrichtung in Bewegung versetzbare Werkzeughalterung (8A, 8B) für ein auf eine Behandlungsstelle einwirkendes Werkzeug (9).

12. Verfahren zur Herstellung eines medizinischen, insbesondere dentalen oder chirurgischen, Instruments oder Instrumententeils (2) nach Anspruch 1, **dadurch gekennzeichnet, dass**
auf zumindest ein Bauteil (8A; 10; 11, 11A; 12, 12A) des Instruments oder Instrumententeils (2), welches ein Substrat (3) mit einer rauen Oberflächenstruktur (4) aufweist, eine glas-keramische Schicht (5) derart aufgebracht oder abgeschieden wird, dass die raue Oberflächenstruktur (4) des Substrats (3) durch die glas-keramische Schicht (5) zumindest nicht vollständig geglättet wird, so dass die raue Oberflächenstruktur (4) des Substrats (3) zumindest teilweise auf die darauf aufgebrachte oder abgeschiedene glas-keramische Schicht (5) übergeht, oder dass die Rauheit der Oberflächenstruktur (4) erhöht wird, wobei die Rauheit der auf dem Substrat (3) aufgebrachten oder abgeschiedenen glas-keramischen Schicht (5) Ra = 0,5 - 1,8 µm und / oder Rz = 3 - 14 µm beträgt.

13. Verfahren zur Herstellung eines medizinischen, insbesondere dentalen oder chirurgischen, Instruments oder Instrumententeils (2) nach Anspruch 12, **dadurch gekennzeichnet, dass**
die auf dem Substrat (3) aufgebrachte oder abgeschiedene glas-keramische Schicht (5) Silizium, insbesondere Siliziumoxid und / oder zumindest eine polymerisierte, vorzugsweise zumindest teilweise organische, Siliziumverbindung, aufweist.

14. Verfahren zur Herstellung eines medizinischen, insbesondere dentalen oder chirurgischen, Instruments oder Instrumententeils (2) nach Anspruch 12 oder 13, **dadurch gekennzeichnet, dass**
die auf dem Substrat (3) aufgebrachte oder abgeschiedene glas-keramische Schicht (5) transparent ist und eine hydrophobe Oberflächeneigenschaft aufweist.

15. Verfahren zur Herstellung eines medizinischen, insbesondere dentalen oder chirurgischen, Instruments oder Instrumententeils (2) nach Anspruch 12, 13 oder 14, **dadurch gekennzeichnet, dass**
die auf dem Substrat (3) aufgebrachte oder abgeschiedene glas-keramische Schicht (5) durch ein CVD (chemical-vapor-deposition) - Verfahren unter Bildung eines Plasmas auf dem Substrat (3) abgeschieden wird.

## Claims

1. A medical, in particular dental or surgical instrument or instrument part (2) having a surface (1A, 1B) comprising a substrate (3) with a rough surface structure (4) and a layer which is applied to or deposited on the substrate (3) in such a way that the rough surface structure (4) of the substrate (3) is at least not completely smoothed by the layer or the roughness is increased, wherein the layer applied to or deposited on the substrate (3) is designed as a glass-ceramic layer (5) and wherein the roughness of the glass-ceramic layer (5) deposited on or applied to the substrate (3) is Ra = 0.5 - 1.8 µm and/or Rz = 3 -14 µm.

2. The medical, in particular dental or surgical instrument or instrument part (2) according to Claim 1, **characterized in that**
the roughness of the substrate (3) is approximately Ra = 0.5 - 1.5 µm and/or Rz = 3 - 12 µm.

3. The medical, in particular dental or surgical instrument or instrument part (2) according to Claim 1 or 2, **characterized in that**
the hardness or mechanical strength of the glass-ceramic layer (5) is 600-800 HV.

4. The medical, in particular dental or surgical instrument or instrument part (2) according to any one of the preceding claims, **characterized by**
a glass-ceramic layer (5) applied to or deposited on the substrate (3) having a hydrophobic surface property.

5. The medical, in particular dental or surgical instrument or instrument part (2) according to any one of Claims 1 - 3, **characterized by**
a glass-ceramic layer (5) applied to or deposited on the substrate (3) having a hydrophilic surface property.

6. The medical, in particular dental or surgical instrument or instrument part (2) according to any one of the preceding claims, **characterized in that**
the layer thickness of the glass-ceramic layer (3) applied to or deposited on the glass-ceramic layer (5) is approximately 1 - 8 µm.

7. The medical, in particular dental or surgical instrument or instrument part (2) according to any one of Claims 1 - 6, **characterized by**
a transparent glass-ceramic layer (5) applied to or deposited on the substrate (3).

8. The medical, in particular dental or surgical instrument or instrument part (2) according to any one of Claims 1 - 7, **characterized in that**
the glass-ceramic layer (5) applied to or deposited on the substrate (3) comprises silicon, in particular silicon oxide and/or at least one polymerized, preferably at least partially organic silicon compound.

9. The medical, in particular dental or surgical instrument or instrument part (2) according to any one of Claims 1 - 8, **characterized in that**
the substrate (3) is formed by a metal, in particular steel or brass, or by a plastic.

10. The medical, in particular dental or surgical instrument or instrument part (2) according to any one of Claims 1 - 9, **characterized in that**
the glass-ceramic layer (5) is applied to or deposited on the substrate (3) by a CVD (chemical-vapor-deposition) method under formation of a plasma.

11. The medical, in particular dental or surgical instrument or instrument part (2) according to any one of the preceding Claims, **characterized by**
a media dispensing device (6, 7) for dispensing a medium in the direction of a treatment site, in particular for dispensing a fluid and/or electromagnetic energy, and/ or a tool mount (8A, 8B) that can be set in motion by a drive device for a tool (9) acting on a treatment site.

12. A method for producing a medical, in particular dental or surgical instrument or instrument part (2) according to Claim 1, **characterized in that**
a glass-ceramic layer (5) is applied to or deposited on at least one component (8A; 10; 11, 11A; 12, 12A) of the instrument or the instrument part (2) which has a substrate (3) with a rough surface structure (4) such, that the rough surface structure (4) of the substrate (3) is at least not completely smoothed by the glass-ceramic layer (5), so that the rough surface structure (4) of the substrate (3) at least partially develops into the applied or deposited glass-ceramic layer (5) or that the roughness of the surface structure (4) is increased, wherein the roughness of the glass-ceramic layer (5) deposited on or applied to the substrate (3) is Ra = 0.5 - 1.8 µm and/or Rz = 3 - 14 µm.

13. The method for producing a medical, in particular dental or surgical instrument or instrument part (2) according to Claim 12, **characterized in that**
the glass-ceramic layer (5) that is applied to or deposited on the substrate (3) comprises silicon, in particular silicon oxide and/or at least one polymerized, preferably at least partially organic silicon compound.

14. The method for producing a medical, in particular dental or surgical instrument or instrument part (2) according to Claim 12 or 13, **characterized in that**
the glass-ceramic layer (5) that is applied to or deposited on the substrate (3) is transparent and has a hydrophobic surface property.

15. The method for producing a medical, in particular dental or surgical instrument or instrument part (2) according to Claim 12, 13 or 14, **characterized in that**
the glass-ceramic layer (5) is applied to or deposited on the substrate (3) by a CVD (chemical-vapor-deposition) method under formation of a plasma.

## Revendications

1. Instrument ou partie d'instrument (2) médical, en particulier dentaire ou chirurgical, avec une surface (1A, 1B) comprenant un substrat (3) avec une structure de surface rugueuse (4) ainsi qu'une couche, laquelle est appliquée ou déposée sur le substrat (3) de manière à ce que la structure de surface rugueuse (4) du substrat (3) n'est au moins pas complètement lissée par la couche ou que la rugosité est accrue, la couche appliquée ou déposée sur le substrat (3) étant réalisée en tant que couche de vitrocéramique (5) et la rugosité de la couche de vitrocéramique (5) appliquée ou déposée sur le substrat (3) étant de Ra = 0,5 - 1,8 µm et/ou Rz = 3 - 14 µm.

2. Instrument ou partie d'instrument (2) médical, en particulier dentaire ou chirurgical selon la revendication 1, **caractérisé en ce que** la rugosité du substrat est de l'ordre de Ra = 0,5 - 1,5 µm et/ou Rz = 3 - 12 µm.

3. Instrument ou partie d'instrument (2) médical, en particulier dentaire ou chirurgical selon la revendication 1 ou 2, **caractérisé en ce que** la dureté ou la résistance mécanique de la couche de vitrocéramique (5) est de 600 - 800 HV.

4. Instrument ou partie d'instrument (2) médical, en particulier dentaire ou chirurgical selon l'une des revendications précédentes, **caractérisé par** une couche de vitrocéramique (5) appliquée ou déposée sur le substrat (3) avec une propriété de surface hydrophobe.

5. Instrument ou partie d'instrument (2) médical, en particulier dentaire ou chirurgical selon l'une des revendications 1 - 3, **caractérisé par** une couche de vitrocéramique (5) appliquée ou déposée sur le substrat (3) avec une propriété de surface hydrophile.

6. Instrument ou partie d'instrument (2) médical, en particulier dentaire ou chirurgical selon l'une des revendications précédentes, **caractérisé en ce que** l'épaisseur de couche de la couche de vitrocéramique (5) appliquée ou déposée sur le substrat (3) est de l'ordre de 1 - 8 µm.

7. Instrument ou partie d'instrument (2) médical, en particulier dentaire ou chirurgical selon l'une des revendications 1 - 6, **caractérisé par** une couche de vitrocéramique transparente (5) appliquée ou déposée sur le substrat (3).

8. Instrument ou partie d'instrument (2) médical, en particulier dentaire ou chirurgical selon l'une des revendications 1 - 7, **caractérisé en ce que** la couche de vitrocéramique (5) appliquée ou déposée sur le substrat (3) présente du silicium, en particulier de l'oxyde de silicium et/ou au moins un composé de silicium polymérisé, de préférence au moins partiellement organique.

9. Instrument ou partie d'instrument (2) médical, en particulier dentaire ou chirurgical selon l'une des revendications 1 - 8, **caractérisé en ce que** le substrat (3) est formé par un métal, en particulier en acier ou laiton, ou bien par un plastique.

10. Instrument ou partie d'instrument (2) médical, en particulier dentaire ou chirurgical selon l'une des revendications 1 - 9, **caractérisé en ce que** la couche de vitrocéramique (5) est appliquée ou déposée sur le substrat (3) grâce à un procédé CVD (chemical-vapor-deposition) en formant un plasma.

11. Instrument ou partie d'instrument (2) médical, en particulier dentaire ou chirurgical selon l'une des revendications précédentes, **caractérisé par** un dispositif de délivrance d'un milieu (6, 7) pour la délivrance d'un milieu en direction d'un emplacement de traitement, en particulier pour la délivrance d'un fluide et/ou d'énergie électromagnétique, et/ou un porte-outil (8A, 8B) pouvant être mis en mouvement par un dispositif d'entraînement pour un outil (9) agissant sur un emplacement de traitement.

12. Procédé pour la fabrication d'un instrument ou partie d'instrument (2) médical, en particulier dentaire ou chirurgical selon la revendication 1, **caractérisé en ce que**
sur au moins un élément (8A ; 10 ; 11, 11A ; 12, 12A) de l'instrument ou de la partie d'instrument (2) qui présente un substrat (3) avec une structure de surface rugueuse (4), on applique ou dépose une couche de vitrocéramique (5) de manière à ce que la structure de surface rugueuse (4) du substrat (3) ne soit au moins pas complètement lissée par la couche de vitrocéramique (5) de sorte que la structure de surface rugueuse (4) du substrat (3) se mélange au moins partiellement avec la couche de vitrocéramique (5) appliquée ou déposée dessus, ou bien **en ce que** la rugosité de la structure de surface (4) est accrue, la rugosité de la couche de vitrocéramique (5) appliquée ou déposée sur le substrat (3) étant de Ra = 0,5 - 1,8 µm et/ou Rz = 3 - 14 µm.

13. Procédé pour la fabrication d'un instrument ou partie d'instrument (2) médical, en particulier dentaire ou chirurgical selon la revendication 12, **caractérisé en ce que**
la couche de vitrocéramique (5) appliquée ou déposée sur le substrat (3) présente du silicium, en particulier de l'oxyde de silicium et/ou au moins un composé de silicium polymérisé, de préférence au moins partiellement organique.

14. Procédé pour la fabrication d'un instrument ou partie d'instrument (2) médical, en particulier dentaire ou chirurgical selon la revendication 12 ou 13, **caractérisé en ce que**
la couche de vitrocéramique (5) appliquée ou déposée sur le substrat (3) est transparente et présente une propriété de surface hydrophobe.

15. Procédé pour la fabrication d'un instrument ou partie d'instrument (2) médical, en particulier dentaire ou chirurgical selon la revendication 12, 13 ou 14, **caractérisé en ce que**
la couche de vitrocéramique (5) appliquée ou déposée sur le substrat (3) est déposée sur le substrat (3) grâce à un procédé CVD (chemical-vapor-deposition) en formant un plasma sur le substrat (3).
